# EUROPÄISCHE PATENTANMELDUNG

(11) **EP 1 686 115 A1**
(43) Veröffentlichungstag der Anmeldung: **02.08.2006**
(21) Anmeldenummer: 05001498.4
(22) Anmeldetag: 26.01.2005
(51) Int. Cl.: C07C 323/52, A61K 31/22

(54) **Organische Säureadditionssalze von Valnemulin**

(71) Anmelder: Novartis AG, 4002 Basel (CH)
(72) Erfinder: Die Erfindernennung liegt noch nicht vor
(74) Vertreter: Liphardt, Bernd

(57) **Zusammenfassung**

Die Erfindung betrifft die Herstellung einer neuen Salzform von Valnemulin, eine Verbindung der Formel I, 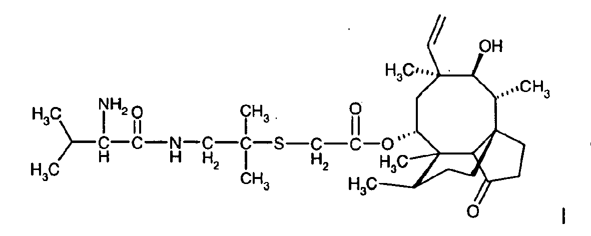 die sich durch ihre gute Kristallinität in erhöhter Reinheit, leichterer technischer Einsetzbarkeit und verbesserter Lagerstabilität auszeichnet.

## Beschreibung

Die vorliegende Erfindung bezieht sich auf die Herstellung einer neuen Salzform von Valnemulin, die sich durch ihre gute Kristallinität in erhöhter Reinheit, leichterer technischer Einsetzbarkeit und verbesserter Lagerstabilität sowohl als purer Wirkstoff als auch verwendet in Formulierungen auszeichnet.

Valnemulin, eine Verbindung der Formel I, ist aus EP 0153277 bekannt; als formuliertes Produkt wird sie unter dem kommerziellen Namen Econor® gehandelt.

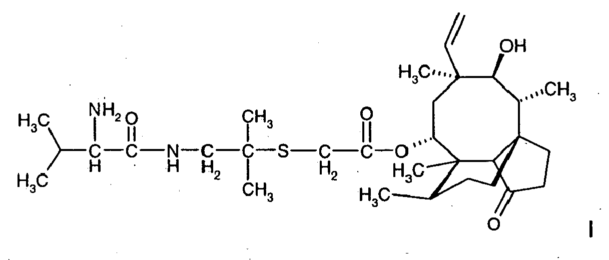

Wie allgemein bekannt ist, hat diese Verbindung antibakterielle Eigenschaften bei z. B. oraler oder parenteraler Administration und wird daher zur Prävention oder Heilung einer Reihe bakterieller Infektionen auf dem Gebiet der Tiergesundheit eingesetzt. Das breite Aktivitätsspektrum umfasst z. B. Streptococcus aronson, Staphylococcus aureus, Mycoplasma arthritidis, Mycoplasma bovigenitalium, Mycoplasma bovimastitidis, Mycoplasma bovirhinis, Mycoplasma sp., Mycoplasma canis, Mycoplasma felis, Mycoplasma fermentans, Mycoplasma gallinarum, Mycoplasma gallisepticum, A. granularum, Mycoplasma hominis, Mycoplasma hyorhinis, Actinobacillus laidlawii, Mycoplasma meleagridis, Mycoplasma neurolyticum, Mycoplasma pneumonia und Mycoplasma hyopneumoniae.

In WO 98/01127 wird ausserdem die ausgezeichnete Aktivität dieser Verbindung gegen einen Krankheitskomplex beschrieben, der unter Verhältnissen auftritt, bei welchen die Tiere in engen, räumlichen Verhältnissen gehalten werden müssen, z. B. zu Transportzwecken, und daher grossem Stress ausgesetzt sind. Die häufigsten Pathogene, die unter diesen Verhältnissen eine entscheidende Rolle spielen, sind Mycoplasma hyopneumoniae, Brachyspira (früher Serpulina oder Treponema) hyodysenteria, Brachyspira pilosicoli, Lawsonia intracellularis, Mycoplasma gallisepticum, Pasteurella multocida, Actinobacillus (Haemophilus) pleuromoniae und Haemophilus parasuis, wobei Atemwegserkrankungen und andere Infektionen oft zusammen auftreten und zu einem komplexen klinischen Bild führen. Sowohl Herdentiere wie auch Haustiere sind betroffen, z. B. Vieh, Schafe und Schweine, Hühner, Hunde und Katzen.

Valnemulin ist als freie Base relativ lagerinstabil und wurde daher entweder in Form des Valnemulin-Cyclodextrin-Komplexes (EP-0,524,63) oder durch Mikrosphäruolen stabilisiert (WO 03/45354), oder in Form der *in situ* hergestellten freien Base (WO 01/41758) oder überwiegend in Form des amorphen Hydrochlorids (EP-0,153,277, WO 98/01127, WO 01/37828) eingesetzt. Die einzige bisher beschrieben Salzform ist das amorphe Hydrochlorid, welches als Reinsubstanz und im formulierten Produkt (Econor® ) lagerstabil ist. Dies gilt allerdings sehr eingeschränkt, wie nachfolgend noch gezeigt wird, im Gemisch mit weiteren Substanzen, insbesondere mit Futter.

Wie unter anderem aus der Europäischen Patentschrift EP0524632 bekannt ist, lassen sich Antibiotika aus der Gruppe der Pleuromutiline, zu denen auch das hier genannte Valnemulin zählt, relativ einfach in Form des wasserlöslichen Hydrochlorids dem Trinkwasser zusetzen. Im Gegensatz dazu erweist es sich aber als schwierig, diese Antibiotika den zu behandelnden Tieren über das Futter zu verabreichen, weil diese Antibiotika durch die Futterbestandteile sehr rasch zersetzt und damit inaktiviert werden. Für die Bereitstellung von Futter-Medikament-Gemischen ist es jedoch wesentlich, dass eine gewisse Lagerstabilität erreicht werden kann, weil sonst eine genaue Dosierung unmöglich ist. Daher wurden schon früher verschiedene Anstrengungen unternommen, um die Stabilität von Valnemulin in Mehlfutter und Futterpellets zu verbessern.

Während man bei Menschen Antibiotika in unterschiedlichsten Applikationsformen, wie Tabletten, Dragees, Emulsionen, Injektionslösungen und dergleichen verabreichen kann, weil man mit der Disziplin und dem Genesungswunsch des menschlichen Patienten rechnen kann, stösst man bei Tieren rasch auf erhebliche praktische Probleme.

Beim Tier muss eine natürliche Bereitschaft vorhanden sein, ein medizinisches Präparat oral aufzunehmen. Natürlich kann man ein einzelnes Tier oder einige wenige Tiere auch zwangsweise behandeln und ihnen ein Antibiotikum derart verabreichen, dass es das Tier schlucken muss, oder man injiziert es. Derartige Zwangsmethoden sind jedoch für grosse Tierbestände nicht akzeptabel, weil sie arbeitsintensiv sind, in jedem Einzelfall den Veterinär erfordern und daher zu hohen Kosten führen. Bei der Gruppentierhaltung sind daher einfache und sichere, vor allem tierschonende Anwendungsformen gesucht, die entweder vom Tier freiwillig aufgenommen werden oder, sofern eine Zwangsmedikation notwendig ist, vom Veterinär oder, sofern erlaubt, vom Tierhalter selbst oder sogar vollautomatisch durchgeführt werden kann, und welche die Kosten in einem verträglichen Rahmen halten.

Eine Methode, die diesen Umständen Rechnung trägt, ist die korrekt dosierte Verabreichung von Antibiotika, eingearbeitet in Tiertrockenfutter, d.h. in Mehlfutter oder Futterpellets.

Haus- und Nutztiere, wie z.B. Schweine, aber auch Kühe, Schafe und Geflügel, werden heutzutage vielfach in Stallungen gehalten, die mit modernsten vollautomatischen Fütterungsanlagen ausgestattet sind. Hierbei wird das Futter, entsprechend Alter und Gewicht des Tieres, in nährstoffbedarfgerechten Mengen z. T. vollautomatisch im Futtertrog bereitgestellt.

In solchen vollautomatischen Anlagen verwendet man vielfach besagte Futterpellets. Dabei handelt es sich um gepresstes, hoch verdichtetes Krafttrockenfutter auf pflanzlicher und/oder tierischer Basis, welches mit Zusätzen wie Aminosäuren, Vitaminen und Mineralstoffen angereichert sein kann. Diese Futterpellets sind nichts anderes als künstliche, rieselfähige, runde oder längliche Körner, Kügelchen oder je nach Herstellungsverfahren auch stäbchenartige Gebilde mit einer einheitlichen, auf Alter und Gewicht der Tiere zugeschnittenen Grösse, die von einigen Millimetern bei Geflügel bis zu ca. einem Zentimeter bei ausgewachsenen Schweinen und Kühen betragen kann. Futterpellets werden von kommerziellen Futtermühlen durch Vermahlen des organischen Ausgangsmaterials, Mischen der Komponenten in gewünschter Zusammensetzung und schliesslich Verpressung zu Pellets angefertigt, in Säcke abgefüllt und beim Tierhalter angeliefert, der sie in die Verteileranlage eingefüllt. Ein wesentlicher Vorteil dieser Pellets besteht in ihrer einfachen Handhabung, welche sich aus ihrer Einheitlichkeit, ihrer Rieselfähigkeit und Lagerstabilität ergibt. Sie lassen sich leicht vollautomatisch abfüllen, dosieren, über Förderbänder oder Rohrleitungen transportieren und an jedes Tier in genau bemessener Portion verabreichen. Pellets nehmen darüber hinaus bedeutend weniger Platz weg als Frischfutter und werden vor allem von den Tieren gerne und problemlos gefressen.

Es bietet sich daher an, diesen Pellets nicht nur Aminosäuren und andere Vitalstoffe, wie Vitamine und Mineralien zuzusetzen, sondern im Bedarfsfall auch Antibiotika. Dies wird in der Praxis bereits durchgeführt, stösst aber bei Vainemulin auf die geschilderten besonderen Schwierigkeiten, die dieser Substanzklasse eigen sind und nachfolgend noch näher erläutert werden.

Es hat sich nämlich gezeigt, dass Vainemulin vor allem in Kontakt mit dem Futtermaterial, insbesondere mit den pflanzlichen und tierischen Bestandteilen, beim Herstellen von Futterpellets ziemlich instabil ist. Dies führt bereits beim Herstellungsvorgang zu erheblichen Verlusten. Bei der Herstellung der Futterpellets wird nämlich das getrocknete organische Ausgangsmaterial tierischer oder pflanzlicher Herkunft vermahlen, mit den Zusatzstoffen, Vitaminen, Spurenelementen und anderen Zusätzen mit der Aktivsubstanz innig vermischt, d.h. weitgehend homogenisiert und anschliessend gegebenenfalls mit ca. 5 bis 10 Gewichtsprozent Wasser angefeuchtet und bei erhöhten Temperaturen im Bereich von über ca. 60 bis 100°C unter Drücken von ca. 1 bis 100 kbar zu Futterpellets verpresst. Kurzzeitige, lokale Temperaturspitzen in der Presse, so genannte Flashes, erreichen sogar kurzfristig 200°C. Die Verweilzeit der Masse in der Presse beträgt im allgemeinen ca. 5 bis 180 Sekunden und hängt unter anderem von der Grösse der Pellets ab.

Während Valnemulin in Form des getrockneten, amorphen Hydrochloridsalzes kurzfristig diese Temperaturen ohne signifikante Zersetzung übersteht und bei Zimmertemperatur sogar einige Monate ohne messbaren Wirkstoffverlust gelagert werden kann, zersetzt sich dieser Wirkstoff relativ rasch unter Druck und im innigen Kontakt mit tierischen oder pflanzlichen Futtermittelbestandteilen und den vorherrschenden erhöhten Temperaturen.

Der Kontakt mit Futterbestandteilen scheint den Abbauprozess geradezu zu katalysieren. Auch wenn man die Phase des erhöhten Drucks und der erhöhten Temperatur so kurz, wie technisch machbar, hält und die fertigen Pellets unmittelbar nach dem Pressvorgang sogleich auf Zimmertemperatur herunterkühlt, geht trotzdem ein Viertel bis ein Drittel des Wirkstoffs, Valnemulin, verloren. Der Wirkstoffverlust führt unweigerlich zu Problemen der korrekten Dosierung am Tier und damit zum Therapieerfolg sowie zu einer beträchtlichen Kostensteigerung beim Endprodukt.

Es hat sich darüber hinaus auch gezeigt, dass das noch intakte Valnemulin in den Pellets deutlich weniger lagerstabil ist als beispielsweise das trockne, amorphe Hydrochlorid. Der Zerfall des Wirkstoffs setzt sich in den fertigen Pellets auch bei Raumtemperatur fort. Bereits nach drei Monaten sinkt der Wirkstoffgehalt auf unter 60%. Diese relative Instabilität hat ferner dazu geführt, dass eine genaue Dosierung des Wirkstoffs in Form von Futterpellets bisher nur für ca. 3 Wochen nach Herstellung der Pellets gewährleistet werden konnte. Die Tierhalter waren deshalb bisher gezwungen, nur relativ frisch hergestellte Pellets einzusetzen. Sie konnten keine langfristige sinnvolle Lagerhaltung betreiben und mussten ca. alle vier bis sechs Wochen den Futtermühlen einen neuen Herstellungsauftrag erteilen, damit ihnen frisches Futter mit einem garantierten Gehalt an Antibiotika zur Verfügung gestellt wurde. Dies ist technisch zwar realisierbar, bedingt aber einen hohen Logistikaufwand und führte dazu, dass die Futtermühlen immer wieder Kleinaufträge durchführen mussten, die nicht unbedingt in ihr Produktionsprogramm passten, was zu enormem Reinigungsaufwand zwischen jeder Charge führte, um Kreuzkontaminationen zu vermeiden und damit einer zusätzlichen Verteuerung der Pellets führte.

Aus den genannten Gründen wurde daher eine Vielzahl von Anstrengungen unternommen, um Valnemulin und andere Vertreter der Klasse der Pleuromutiline so zu stabilisieren, dass sie die erhöhten Temperaturen und Pressdrücke bei der Pelletherstellung ohne Verlust an Aktivsubstanz überstehen und auch in Form der hergestellten Pellets eine praxisgerechte Langzeitstabilität aufweisen.

Zu diesen erfolglosen Versuchen gehören z.B. (1) die Verringerung der Wirkstoffoberfläche durch Verpressung zu Körnern, wobei unterschiedlichste Korngrössen ausprobiert wurden; (2) das Versiegeln besagter Wirkstoffkörner mit unterschiedlichsten Schutzschichten, beispielsweise mit Gelatine oder verschiedenen Zuckern und Lacken; (3) der Einschluss des Wirkstoffs in poröse Materialien, wie z.B. in unterschiedlichen Zellulosen, Stärken, Kieselsäuren oder Zeolithen mit und ohne zusätzliche Schutzschichten; oder (4) die chemische Modifikation des makrozyklischen Grundgerüsts des Wirkstoffs. Die chemische Modifikation führte zwar in einigen Fällen zu einer verbesserten Stabilität des Moleküls an sich, aber gleichzeitig zu Wirkungsverlust.

In EP0524632 hat man durch Komplexierung mit Cyclodextrin versucht, die Lagerstabilität in Trockenfutter zu erhöhen, was auch teilweise gelungen ist.

Ein anderer, erfolgreicherer Versuch zur Stabilisierung von Pleuromutilinen wie dem hier genannten Valnemulin wird in WO 03/45354 beschrieben. Dabei wird der Wirkstoff in einem speziellen Verfahren in Mikrosphäruolen eingeschlossen; diese Mikrosphäruolen werden dann in das Tiertrockenfutter eingebracht und bei erhöhtem Druck und erhöhter Temperatur zu Futterpellets verpresst. Dieses Verfahren ist jedoch technisch sehr aufwändig und führt zu einer erheblichen Verteuerung der Futterpellets.

Andere Patentreferenzen, wie z.B. WO 01/41758, beschreiben die Herstellung von Injektionslösungen und die damit verbundenen, technischen Schwierigkeiten: So beobachtet man bei Injektionen unter anderem unerwünschte Nebeneffekte, die von milden Hautirritationen bis hin zu schlecht heilenden Nekrosen reichen können. Dies ist auch einer der Gründe, weshalb Valnemulin seither hauptsächlich oral eingesetzt wurde. Von Anwendern wird jedoch manchmal auch beklagt, dass wässrige Lösungen keine Depotwirkung zeigen. Ein weiteres Problem besteht auch darin, dass Valnemulin in freier Form, als so genannte Valnemulin-Base, ausserordentlich instabil ist und daher als amorphes Hydrochlorid-Salz hergestellt und seither vorzugsweise in dieser Form zur Behandlung von bakteriellen Infektionen eingesetzt wurde. Injektionsformen mit verbesserter Verträglichkeit werden in obiger WO 01/41758 beschrieben. Zubereitungen mit amorphem Valnemulin-Hydrochlorid führen vereinzelt zu Palatabilitätsproblemen, wenn sie oral, insbesondere in flüssiger Form, angeboten werden.

Es ist damit offensichtlich, dass nach wie vor ein grosser Bedarf an einer Anwendungsform von Valnemulin besteht, die sich einfach und damit kostengünstig herstellen, dank der genauen Vermischbarkeit mit Futtermitteln gut und wiederholbar dosieren lässt und zu der gewünschten Stabilität bei der Herstellung und Lagerung von Trockentierfutter führt.

In einigen Patentreferenzen werden bereits Salze von Valnemulin mit organischen Säuren erwähnt oder sogar namentlich genannt. So werden beispielsweise in EP-0,153,277 konkret das Hyrogenfumarat, das Fumarat und das Naphthalin-1,5-sulphonat von Valnemulin namentlich genannt. Wenn man diese Referenz jedoch studiert, stellt man fest, dass es sich um rein hypothetische Substanzen handelt, da weder über deren Herstellung noch irgendwelche chemischen oder biologischen Daten berichtet wird. Das einzige in EP-0,153,277 offenbarte Salz ist amorphes Hydrochlorid. Demzufolge sind Salze von Valnemulin mit organischen Säuren, insbesondere in kristalliner Form, nach wie vor neu. Selbst kristallines Hydrochlorid wird nirgendwo beschrieben.

Es ist nunmehr überraschenderweise gelungen, mit bestimmten Salzen von organischen Säuren eine Anwendungsform von Valnemulin bereitzustellen, welche die genannten Stabilitäts- und Palatabilitätsvorteile in sich vereinigt und sich kostengünstig herstellen lässt, wobei im Rahmen der vorliegenden Erfindung insbesondere die kristallinen Salze mit organischen Säuren bevorzugt werden, weil deren Stabilität gegenüber den amorphen Salzen weitaus erhöht ist und die kristallinen Salze darüber hinaus, bei oraler Verabreichung, von Tieren weit besser akzeptiert werden. Palatabilität bei oralen Verabreichungsformen in der Tiermedizin kann über Erfolg und Misserfolg bei der Behandlung entscheiden. Es ist deshalb auch ein Ziel der vorliegenden Anmeldung, eine Anwendungsform mit verbesserter Palatabilität zu Verfügung zu stellen.

Die vorliegende Erfindung löst diese Aufgabe in optimaler Weise, indem Valnemulin mit organischen Säuren zu einem Säureadditionssalz umgesetzt wird, welche überraschend hohe Kristallinität und Lagerstabilität aufweisen. Prinzipiell eignen sich alle physiologisch verträglichen, organischen Säuren. Beispiele geeigneter Säuren sind Monocarbonsäuren wie Ameisensäure, Essigsäure, Propionsäure, Ascorbinsäure, Glykolsäure, Milchsäure, Brenztraubensäure oder Mandelsäure, aber auch Dicarbonsäuren wie Oxalsäure, Malonsäure, Bernsteinsäure, Asparaginsäure, Glutaminsäure, Glutarsäure, Adipinsäure, Pimelinsäure, Korksäure, Maleinsäure, Fumarsäure, Phthalsäure, Isophthalsäure, Terephthalsäure, Äpfelsäure oder Weinsäure, oder auch Tricarbonsäuren wie z. B. Zitronensäure. Generell sind die enantiomerenreinen Mono- und Dicarbonsäuren vorzuziehen. Insbesondere eignen sich D-Weinsäure und Fumarsäure, ganz speziell D-Weinsäure.

Der Vorteil der gemäss dieser Erfindung hergestellten Säureadditionssalze liegt auch in der guten Kristallinität, welche zu einer hohen Reinheit und damit Anwendungssicherheit der daraus hergestellten Anwendungsformen führt.

Ein generelles Verfahren zur Herstellung reiner Valnemulinsalze mit organischen Säuren besteht z. B. darin, dass man
a) rohes Valnemulin-Hydrochlorid durch Umsetzung mit einer Base in die freie Valnemulin-Base umsetzt,
b) gegebenenfalls die freie Base mit einem organischen Lösungsmittel extrahiert und nach üblichen Verfahren isoliert,
c) die Valnemulin-Base in einem organischen Lösungsmittel oder einem Gemisch aus organischen Lösungsmitteln und gegebenenfalls Wasser mit einer organischen Säure gegebenenfalls bei erhöhter Temperatur umsetzt; und
d) gegebenenfalls nach Zugabe einiger Impfkristalle, gegebenenfalls durch langsames Abkühlen der Reaktionslösung das Valnemulin-Säureadditionssalz auskristallisieren lässt.

Bei zwei- oder dreibasischen organischen Säuren können äquimolare oder äquinormale Mengen der Säure mit der Valnemulin-Base umgesetzt werden, wobei äquimolare Mengen bevorzugt werden.

Geeignete Basen zur Freisetzung der Valnemulin-Base sind z. B. Alkalimetall- oder Erdalkalimetallhydroxide, -hydride, -amide, -alkanolate, -acetate oder -carbonate, Alkylamine, Alkylendiamine, gegebenenfalls N-alkylierte, gegebenenfalls ungesättigte, Cycloalkylamine, Ammoniumhydroxide sowie carbocyclische Amine. Beispielhaft seien Natriumhydroxid, -hydrid, -amid, -methanolat, -acetat, -carbonat, Kalium-tert.-butanolat, -hydroxid, -carbonat, -hydrid, Calciumhydrid, Triethylamin, Diisopropylethylamin, Triethylendiamin, Cyclohexylamin, N-Cyclohexyl-N,N-dimethylamin, Benzyltrimethylammoniumhydroxid sowie 1,5-Diazabicyclo[5.4.0]undec-5-en (DBU) genannt. Bevorzugt sind Alkalimetallhydroxide, insbesondere Natriumhydroxid.

Geeignete Lösungsmittel zur Extraktion der freien Valnemulin-Base sind aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäuremethylester, Essigsäureethylester, Essigsäureisopropylester oder Essigsäurebutylester; oder Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan, oder Gemische davon. Bevorzugt sind Ether, insbesondere tert.-Butylmethylether.

Geeignete Lösungsmittel zur Umsetzung der Valnemulin-Base mit einer organischen Säure sind aromatische, aliphatische und alicyclische Kohlenwasserstoffe und Halogenkohlenwasserstoffe, wie Benzol, Toluol, Xylol, Mesitylen, Tetralin, Chlorbenzol, Dichlorbenzol, Brombenzol, Petrolether, Hexan, Cyclohexan, Dichlormethan, Trichlormethan, Tetrachlormethan, Dichlorethan, Trichlorethen oder Tetrachlorethen; Ester, wie Essigsäuremethylester, Essigsäureethylester, Essigsäureisopropylester oder Essigsäurebutylester; Ether, wie Diethylether, Dipropylether, Diisopropylether, Dibutylether, tert.-Butylmethylether, Ethylenglykolmonomethylether, Ethylenglykolmonoethylether, Ethylenglykoldimethylether, Dimethoxydiethylether, Tetrahydrofuran oder Dioxan; Ketone, wie Aceton, Methylethylketon oder Methylisobutylketon; Alkohole, wie Methanol, Ethanol, Propanol, Isopropanol, Butanol, Ethylenglykol oder Glycerin; Amide, wie N,N-Dimethylformamid, N,N-Diethylformamid, N,N-Dimethylacetamid, N-Methylpyrrolidon oder Hexamethylphosphorsäuretriamid; oder Nitrile, wie Acetonitril oder Propionitril; und Sulfoxide, wie Dimethylsulfoxid, oder Gemische davon mit und ohne Wasser.

Bevorzugt sind Ester und Ketone sowie deren Gemische mit Wasser, insbesondere Gemische aus Essigsäureethylester, Aceton und Wasser. Ganz besonders bevorzugt sind Lösungsmittelgemische aus ca. 70 Vol.% bis ca. 90 Vol.% Essigsäureethylester, ca. 5 Vol.% bis ca. 25 Vol.% Aceton und ca. 0 bis ca. 5 Vol.% Wasser, ganz speziell Gemische aus ca. 75 Vol.% bis ca. 80 Vol.% Essigsäureethylester, ca. 20 Vol.% bis ca. 25 Vol.% Aceton und ca. 1 bis ca. 2 Vol.% Wasser.

Im Folgenden sind beispielhafte Herstellungsverfahren von Vainemulin-Säureadditionssalzen aus der Umsetzung von freiem Valnemulin oder seinem Hydrochlorid mit organischen Säuren beschrieben. Alle Temperaturen sind in Celsiusgraden angegeben.

### Herstellungsbeispiele

### Beispiel 1: Herstellung von Valnemulin-D-Hydrogentartrat durch Umsalzung

a) In 300 ml auf 30-35°C erwärmtes Wasser werden unter Rühren 30,1 g Valnemulin-Hydrochlorid eingetragen. Danach werden 150 ml tert. Butylmethylether zugefügt und der pH mit ca. 5 ml 10 n Natronlauge auf 8 bis 9 gestellt. Nach 5-minütigem Rühren bei 30-35°C wird die organische Phase abgetrennt und zweimal mit je 100 ml Wasser ausgewaschen. Anschliessend wird das organische Lösungsmittel unter Normaldruck abdestilliert.
b) Der Eindampfrückstand, bestehend aus ungereinigtem Valnemulin, wird bei ca. 40°C in einer vorbereiteten Lösung aus 50 ml Ethanol und 7,5 g D-Weinsäure aufgelöst und das Gemisch mit 350 ml Methylethylketon verrührt. Beim Erreichen einer Temperatur von ca. 35°C werden unter intensivem Rühren Impfkristalle zugegeben, wodurch die Titelverbindung langsam auskristallisiert. Die Suspension wird während ca. 8 Stunden unter passiver Kühlung weiter gerührt. Anschliessend wird das Produkt filtriert, mit Methylethylketon gewaschen und bei 50°C getrocknet. Man erhält so die Titelverbindung als weisse Kristalle vom Smp. 130°C.

### Beispiel 2: Herstellung von Valnemulin-D-Hydrogentartrat aus freier Base

In 240 ml einer Lösungsmittel-Mischung, bestehend aus 78% Essigsäureethylester, 20,5% Aceton und 1,5% Wasser, werden bei 65°C 23,5 g Valnemulin gelöst, anschliessend 6,9 g D-Weinsäure zugegeben und das Gemisch gerührt, bis eine klare Lösung entstanden ist. Unter weiterem Rühren werden 25 mg Impfkristalle zugefügt, wodurch nach ca. 10 Minuten die Kristallisation beginnt. Die Suspension wird eine weitere Stunde bei der Siedetemperatur gerührt und danach innert 2 Stunden auf Raumtemperatur gekühlt. Das ausgefallene Produkt wird filtriert und über Nacht am Vakuum bei 50°C getrocknet. Man erhält so die Titelverbindung als weisse Kristalle vom Smp. 172°C.

### Beispiel 3: Herstellung von Valnemulin-Hydrogenfumarat aus freier Base

In 220 ml einer Lösungsmittel-Mischung, bestehend aus 78% Essigsäureethylester, 20,5% Aceton und 1,5% Wasser, werden bei 40°C 59,3 g Valnemulin gelöst, anschliessend 12,1 g Fumarsäure zugegeben und das Gemisch gerührt, bis eine klare Lösung entstanden ist. Die Mischung wird danach auf 30°C gekühlt und unter Rühren 0,5 g Impfkristalle zugefügt.

Anschliessend wird das Gemisch während 3 Stunden bei 30°C weiter gerührt und dann über Nacht auf Raumtemperatur kühlen gelassen. Danach wird das Gemisch weitere 2 Stunden bei 0°C gerührt. Die kalte Suspension wird schliesslich filtriert, der Rückstand mit Essigsäureethylester gewaschen und über Nacht am Vakuum bei 50°C getrocknet. Man erhält so die Titelverbindung als weisse Kristalle vom Smp. 132°C.

### Stabilitätsuntersuchungen

Zur Untersuchung der Stabilität der Säureadditionssalze von Valnemulin im Tierfutter wird die berechnete Menge des Säureadditionssalzes (entsprechend einer Zieldosis von 100ppm) zu ca. 4 kg weizenbasiertem Tierfutter zugefügt und in einem Labor-Hochgeschwindigkeitsmixer während 60 sek. zu einem ersten Premix (PM1) vermischt. Die ca. 4 kg Premix PM1 werden anschliessend in einen horizontalen Mischer transferiert und mit 21 kg desselben Futters erneut 6 min. zu einem weiteren Premix (PM2) vermischt. Die ca. 25 kg Premix PM2 werden in einen vertikalen Mischer übergeführt und mit weiteren 175 kg desselben Tierfutters während 8 min. innig vermischt, wodurch man eine homogene, behandelte Mischung erhält, welche Valnemulin-Konzentrationen enthält, die der Zieldosis von 100 ppm entsprechen.

Vor der Weiterverarbeitung werden Proben von je ca. 100 g vom oberen, mittleren und unteren Teil der Medizinalfuttermischung entnommen, um deren Homogenität zu überprüfen. Ausserdem werden weitere Proben in zufälliger Weise zur Verwendung in Stabilitätstests gezogen.

Das fertig gestellte, behandelte Medizinalfutter ist nun bereit zur Pelletierung. Zu diesem Zweck wird es durch eine Konditionierungskammer geführt, worin 3 kg Wasser und gesättigter Wasserdampf beigefügt werden, um die Pelletierungstemperatur auf 75°C bis 85°C einzustellen. Die 200 kg konditionierten Medizinalfutters werden innert ca. 20 min. kontinuierlich in die Pelletierungspresse der Futtermühle eingespiesen. Danach werden die Pellets chargenweise in einem kontinuierlichen Luftstrom getrocknet und gekühlt.

Zur Überprüfung der Homogenität des pelletierten Medizinalfutters werden mehrere Proben von je ca. 100 g zu Beginn, in der Mitte und am Ende des Pelletierungsprozesses entnommen. Zusätzlich werden Proben in zufälliger Abfolge aus jeder Charge für die Lagerstabilitätsuntersuchungen gezogen.

Für die nachfolgenden Beispiele betragen die Mengen an Wirkstoff pro 200 kg Tierfutter jeweils 20 g Valnemulin-Hydrochlorid, Valnemulin-D-Hydrogentartrat, respektive Valnemulin-Hydrogenfumarat, was 100 ppm an Valnemulin im Futter entspricht.

In der folgenden Tabelle 1 werden beispielhaft die Stabilitäten verschiedener Säureadditionssalze von Valnemulin beim Pelletierungsvorgang des Tierfutters verglichen.

**Tabelle 1: Verlust von Wirksubstanz beim Pelletierungsvorgang bei 75°C**

| Wirksubstanz | Mittlerer Verlust in % |
|---|---|
| Valnemulin-Hydrochlorid | 11.6 |
| Valnemulin-D-Hydrogentartrat | 0.3 |
| Valnemulin-Hydrogenfumarat | 0.2 |

Diese Daten zeigen die ausserordentlich hohe Stabilität der Additionssalze von Valnemulin mit organischen Säuren im Vergleich zum bekannten Hydrochlorid.

## Patentansprüche

1. Salze, bestehend aus Valnemulin der Formel I 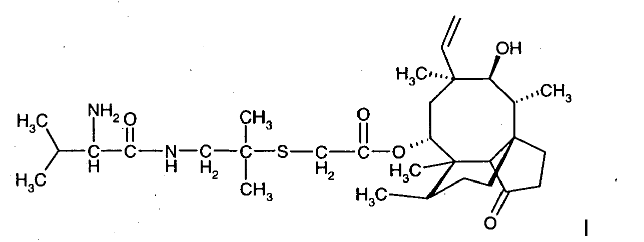 und organischen Säuren.

2. Salze gemäss Anspruch 1, **dadurch gekennzeichnet, dass** die organischen Säuren ausgewählt sind aus der Gruppe, bestehend aus Monocarbonsäuren, Dicarbonsäuren und Tricarbonsäuren.

3. Salze gemäss Anspruch 1, **dadurch gekennzeichnet, dass** die organischen Säuren ausgewählt sind aus der Gruppe, bestehend aus Ameisensäure, Essigsäure, Propionsäure, Ascorbinsäure, Glykolsäure, Milchsäure, Brenztraubensäure, Mandelsäure, Oxalsäure, Malonsäure, Bernsteinsäure, Asparaginsäure, Glutaminsäure, Glutarsäure, Adipinsäure, Pimelinsäure, Korksäure, Maleinsäure, Fumarsäure, Phthalsäure, Isophthalsäure, Terephthalsäure, Äpfelsäure, Weinsäure und Zitronensäure.

4. Salze gemäss Anspruch 1, **dadurch gekennzeichnet, dass** die organischen Säuren ausgewählt sind aus der Gruppe, bestehend aus enantiomerenreinen Mono- und Dicarbonsäuren.

5. Salze gemäss Anspruch 1, **dadurch gekennzeichnet, dass** die organischen Säuren ausgewählt sind aus der Gruppe, bestehend aus D-Weinsäure und Fumarsäure.

6. Salz gemäss Anspruch 1, **dadurch gekennzeichnet, dass** die organische Säure D-Weinsäure ist.

7. Salze gemäss einem der Ansprüche 1 bis 6, **dadurch gekennzeichnet, dass** besagte Salze in kristalliner Form vorliegen.

8. Verfahren zur Herstellung der Salze gemäss einem der Ansprüche 1 bis 7, **dadurch gekennzeichnet, dass** man
a) rohes Valnemulin-Hydrochlorid durch Umsetzung mit einer Base in die freie Valnemulin-Base umsetzt,
b) gegebenenfalls die freie Base mit einem organischen Lösungsmittel extrahiert und nach üblichen Verfahren isoliert,
c) die Valnemulin-Base in einem organischen Lösungsmittel oder einem Gemisch aus organischen Lösungsmitteln und gegebenenfalls Wasser mit einer organischen Säure gegebenenfalls bei erhöhter Temperatur umsetzt; und
d) gegebenenfalls nach Zugabe einiger Impfkristalle, gegebenenfalls durch langsames Abkühlen der Reaktionslösung das Valnemulin-Säureadditionssalz auskristallisieren lässt.

9. Verfahren gemäss Anspruch 8, **dadurch gekennzeichnet, dass** die Extraktion der Valnemulin-Base in einem Ether erfolgt.

10. Verfahren gemäss Anspruch 8, **dadurch gekennzeichnet, dass** die Extraktion der Valnemulin-Base in tert.-Butylmethylether erfolgt.

11. Verfahren gemäss Anspruch 8, **dadurch gekennzeichnet, dass** die Umsetzung der Valnemulin-Base mit einer organischen Säure in einem Lösungsmittel aus der Gruppe der Alkohole und Ketone erfolgt.

12. Verfahren gemäss Anspruch 11, **dadurch gekennzeichnet, dass** das Lösungsmittel Ethanol und Methylethylketon ist.

13. Verfahren gemäss Anspruch 8, **dadurch gekennzeichnet, dass** die Umsetzung der Valnemulin-Base mit einer organischen Säure in einem Lösungsmittelgemisch aus der Gruppe der Ester und der Gruppe der Ketone und Wasser erfolgt.

14. Verfahren gemäss Anspruch 13, **dadurch gekennzeichnet, dass** das Lösungsmittelgemisch aus Essigsäureethylester, Aceton und Wasser besteht.

15. Verfahren gemäss Anspruch 13, **dadurch gekennzeichnet, dass** das Lösungsmittelgemisch aus ca. 70 Vol.% bis ca. 90 Vol.% Essigsäureethylester, ca. 5 Vol.% bis ca. 25 Vol.% Aceton und ca. 0 bis ca. 5 Vol.% Wasser besteht.

16. Verfahren gemäss Anspruch 13, **dadurch gekennzeichnet, dass** das Lösungsmittelgemisch aus ca. 75 Vol.% bis ca. 80 Vol.% Essigsäureethylester, ca. 20 Vol.% bis ca. 25 Vol.% Aceton und ca. 1 bis ca. 2 Vol.% Wasser besteht.

17. Verwendung eines Salzes gemäss einem der Ansprüche 1 bis 6 zur Herstellung eines Medikaments zur Behandlung bakterieller Infektionen von warmblütigen Tieren.

18. Verwendung eines Salzes gemäss einem der Ansprüche 1 bis 6 als Tierfutterzusatz.

19. Tierfutter-Pellets, **dadurch gekennzeichnet, dass** sie eine wirksame Menge mindestens eines Salzes gemäss einem der Ansprüche 1 bis 6 enthalten.

20. Verwendung eines Salzes gemäss einem der Ansprüche 1 bis 6 als Trinkwasserzusatz.
